# EUROPEAN PATENT SPECIFICATION

(11) **EP 2 540 211 B1**
(45) Date of publication and mention of the grant of the patent: **23.10.2013**
(21) Application number: 11828637.6
(22) Date of filing: 10.08.2011
(51) Int. Cl.: A61B 1/00, A61B 17/29

(54) **ENDOSCOPE DEVICE**
ENDOSKOPVORRICHTUNG
DISPOSITIF ENDOSCOPIQUE

(30) Priority: 28.09.2010 JP 2010217904
(43) Date of publication of application: 02.01.2013
(73) Proprietor: OLYMPUS MEDICAL SYSTEMS CORP., Tokyo 151-0072 (JP)
(72) Inventor: NAITO, Kimihiko, Tokyo 192-8512 (JP)
(74) Representative: Schicker, Silvia
(86) International application number: PCT/JP2011/068303
(87) International publication number: WO 2012/043076

(56) References cited:
- JP-A- 7 116 104
- JP-A- 10 201 704
- JP-A- 10 201 704
- JP-A- 2000 325 303
- JP-A- 2006 181 180
- JP-A- 2006 230 837
- JP-A- 2009 056 130
- JP-U- 57 079 503
- US-A1- 2005 096 694
- US-A1- 2007 219 550
- US-A1- 2010 022 827
- US-A1- 2010 056 863

## Description

### Technical Field

The present invention relates to an endoscopic device including an endoscope and a treatment instrument inserted into a treatment instrument insertion channel of the endoscope.

### Background Art

Patent Literature 1 discloses an endoscopic device including an endoscope to which two treatment instrument insertion channels provided, and treatment instruments each of which is inserted into corresponding treatment instrument insertion channel. In this endoscopic device, when giving a treatment with respect to a diseased part using a treatment instrument, each treatment instrument protrudes from a distal end of the endoscope toward a distal direction side.

Further, in the endoscopic device, a treatment instrument in which a treatment instrument bending section configured to perform a bending action is provided may be used in some cases. In the treatment instrument including the treatment instrument bending section, when a bending operation wire extended in the treatment instrument is pulled or loosened, the treatment instrument bending section is bent. The bending operation wire is formed by twisting, for example, strands.

Document US 2010/056863 A1 discloses an endoscopic device comprising a treatment instrument insertion section which includes a treatment instrument bending section configured to bend, which is configured to be inserted into the body cavity through a treatment instrument insertion channel extended in an endoscope insertion section along longitudinal directions, a treatment instrument operation section which is provided to the proximal direction side of the treatment instrument insertion section and a bending operation wire which has one end connected to the treatment instrument bending section, and the other end connected to the treatment instrument operation section, and which is configured to bend the treatment instrument bending section when pulled or loosened by an operation in the treatment instrument operation section, wherein the bending operation wire includes a rigidity variable wire which includes a first wire portion, a second wire portion provided to the proximal direction side of the first wire portion and having a rigidity higher than that of the first wire portion, and a wire joint portion in which the first wire portion and the second wire portion are joined.

### Citation List

### Patent Literature

Patent Literature 1: Jpn. Pat. Appln. KOKAI Publication No. 2000-37348

### Summary of Invention

### Technical Problem

In the treatment instrument including the treatment instrument bending section configured to be bent by pulling or loosening the bending operation wire, when the bending operation wire is pulled, the bending operation wire is elongated. When the bending operation wire is elongated, a bending amount of the treatment instrument bending section with respect to a pulling amount of the bending operation wire varies. Therefore, the operability in the bending operation of the treatment instrument bending unit in the treatment instrument is deteriorated.

For example, it can be considered that, when a diameter of each strand forming the bending operation wire is increased, or when the bending operation wire is formed of one strand in a state that the diameter of the bending operation wire is evenly maintained, the rigidity of the bending operation wire is improved, and the elongation of the bending operation wire is reduced. However, in general, an endoscope of an endoscopic device includes an endoscope bending section configured to perform a bending action. Therefore, when the rigidity of the bending operation wire is increased, at the time of inserting a treatment instrument into the endoscope to be used, the bending operation of the endoscope bending section is hard to perform.

The present invention has been developed in view of the above circumstances, and an object thereof is to provide an endoscopic device that improves the operability in a bending operation of a treatment instrument bending section without deteriorating the operability in the bending operation of the endoscope bending section.

### Solution to Problem

To solve above mentioned problems, according to one aspect of the invention, an endoscopic device as claimed in claim 1 includes: an endoscope insertion section which includes an endoscope bending section configured to bend, and an endoscope flexible tube section provided to a proximal direction side of the endoscope bending section, and which is configured to be inserted into a body cavity; a treatment instrument insertion section which includes a treatment instrument bending section configured to bend, and which is configured to be inserted into the body cavity through a treatment instrument insertion channel extended in the endoscope insertion section along longitudinal directions; a treatment instrument operation section which is provided to the proximal direction side of the treatment instrument insertion section; and a bending operation wire which has one end connected to the treatment instrument bending section, and the other end connected to the treatment instrument operation section, and which is configured to bend the treatment instrument bending section when pulled or loosened by an operation in the treatment instrument operation section, wherein the bending operation wire includes a rigidity variable wire which includes a first wire portion, a second wire portion provided to the proximal direction side of the first wire portion and having a rigidity higher than that of the first wire portion, and a wire joint portion in which the first wire portion and the second wire portion are joined, the wire joint portion being configured to be placed to the proximal direction side of a proximal end of the endoscope bending section at a treatment in which a proximal end of the treatment instrument bending section is placed to a distal direction side of a distal end of the endoscope insertion section.

### Advantageous Effects of Invention

According to the present invention, an endoscopic device, which improves the operability in a bending operation of a treatment instrument bending section without deteriorating the operability in the bending operation of the endoscope bending section, can be provided.

### Brief description of Drawings

FIG. 1 is a perspective view showing an example of an endoscopic device not according to the present invention;
FIG. 2 is a schematic view showing the endoscopic device according to the first example;
FIG. 3 is a schematic view showing a part of the endoscopic device on a distal direction side according to the first example;
FIG. 4 is a cross-sectional view taken along line IV-IV in FIG. 3;
FIG. 5 is a cross-sectional view taken along line V-V in FIG. 3;
FIG. 6 is a schematic view showing a bending operation wire of a treatment instrument in an endoscopic device according to a modification of the first example;
FIG. 7 is a cross-sectional view taken along line VII-VII in FIG. 6;
FIG. 8 is a cross-sectional view taken along line VIII-VIII in FIG. 6;
FIG. 9 is a schematic view showing a part of a treatment instrument on a distal direction side in an endoscopic device according to an embodiment of the present invention;
FIG. 10 is a cross-sectional view taken along line X-X in FIG. 9;
FIG. 11 is a cross-sectional view taken along line 11-11 in FIG. 9;
FIG. 12 is a schematic view showing a part of an endoscopic device on a distal direction side according to an embodiment of the present invention;
FIG. 13 is a schematic view showing a state of the part of the endoscopic device on the distal direction side at the time of a treatment using a first treatment instrument and a second treatment instrument according to the embodiment;
FIG. 14 is a schematic view showing a part of the treatment instrument on the distal direction side in the endoscopic device according to the embodiment;
FIG. 15 is a cross-sectional view taken along line 15-15 in FIG. 14; and
FIG. 16 is a cross-sectional view taken along line 16-16 in FIG. 14.

### Description of Embodiments

### (First Embodiment)

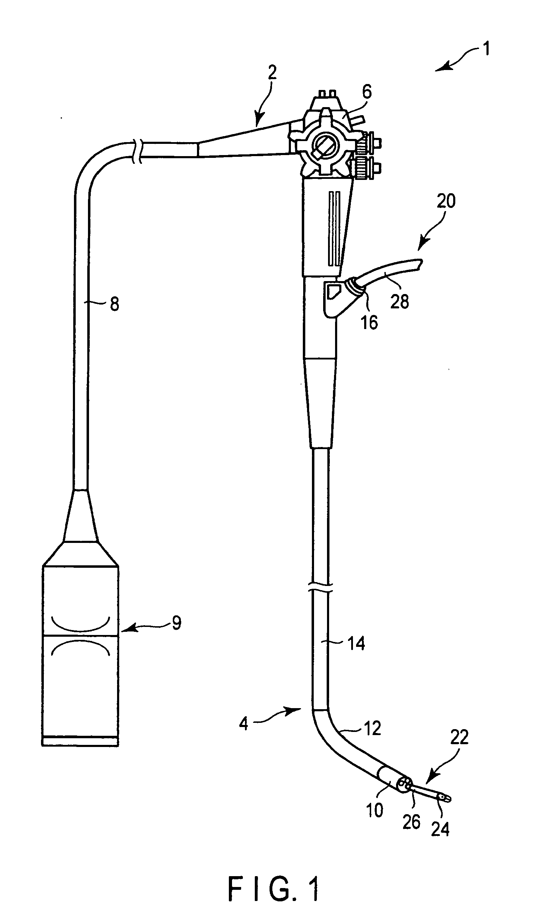

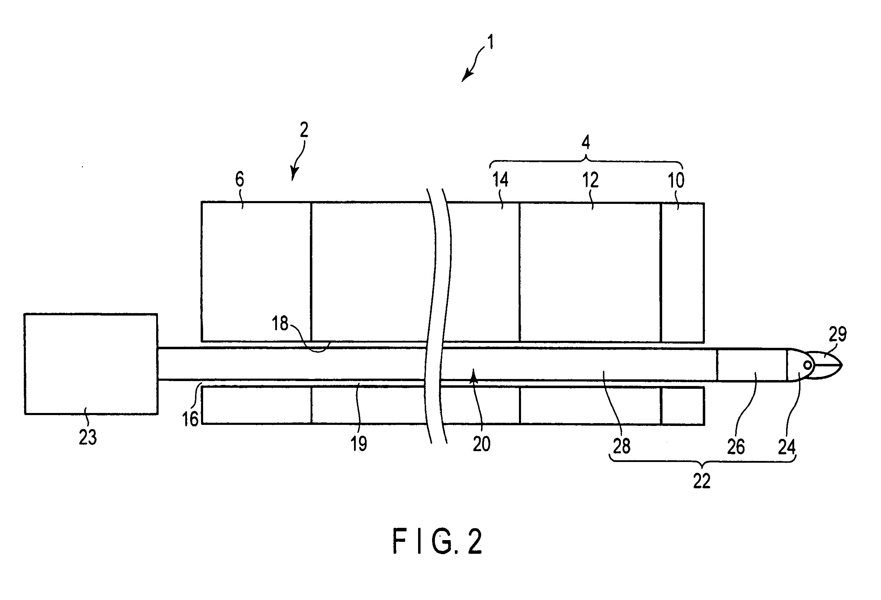

A first embodiment not according to the present invention will now be described with reference to FIG. 1 to FIG. 5. FIG. 1 and FIG. 2 are views showing an endoscopic device 1 according to this embodiment. As shown in FIG. 1, the endoscopic device 1 includes an endoscope 2 configured to image a subject such as a diseased part. The endoscope 2 includes an endoscope insertion section 4 configured to be inserted into a body cavity, and an endoscope operation section 6 provided to a proximal direction side of the endoscope insertion section 4. One end of a universal cord 8 is connected to the endoscope operation section 6. The other end of the universal cord 8 is connected to an image processing unit, an illumination power supply unit (which are not shown), or the like through a scope connector 9.

As shown in FIG. 1 and FIG. 2, the endoscope insertion section 4 includes a distal end hard section 10 which is provided on a most distal direction side and hard, an endoscope bending section 12 which is provided to the proximal direction side of the distal end hard section 10 and is configured to perform a bending action, and an endoscope flexible tube section 14 which is provided to the proximal direction side of the endoscope bending section 12, and which is elongated and flexible. A treatment instrument insertion opening 16 is provided on the endoscope operation section 6 in the endoscope 2. A channel defining inner peripheral surface portion 18 is extended from the treatment instrument insertion opening 16 to the distal end hard section 10 through the endoscope flexible tube section 14 and the endoscope bending section 12 along longitudinal directions. A treatment instrument insertion channel 19 is defined by the channel defining inner peripheral surface portion 18. That is, the treatment instrument insertion channel 19 is extended in the endoscope insertion section 4 along the longitudinal directions.

A treatment instrument 20 is configured to be inserted into the treatment instrument insertion channel 19 of the endoscope 2 to be movable forward and backward in the longitudinal directions. At the time of a treatment using the treatment instrument 20, the treatment instrument 20 is inserted into the treatment instrument insertion channel 19 from the treatment instrument insertion opening 16 and protrudes from a distal end of the endoscope insertion section 4 (the distal end hard section 10) toward the distal direction side. The treatment instrument 20 includes a treatment instrument insertion section 22 configured to be inserted into a body cavity through the treatment instrument insertion channel 19, and a treatment instrument operation section 23 which is provided to the proximal direction side of the treatment instrument insertion section 22. The treatment instrument -insertion section 22 includes a distal end treatment section 24 which is provided on the most distal direction side and is configured to give a treatment for a diseased part, a treatment instrument bending section 26 which is provided to the proximal direction side of the distal end treatment section 24 and is configured to perform a bending action, and a treatment instrument tubular section 28 which is provided to the proximal direction side of the treatment instrument bending section 26 and extended in the longitudinal directions. With an operation in the treatment instrument operation section 23, the treatment instrument bending section 26 carries out the bending action. At the time of a treatment using the treatment instrument 20, a proximal end of the treatment instrument bending section 26 is placed to the distal direction side of the distal end of the endoscope insertion section 4 (the distal end hard section 10). As a result, the treatment instrument bending section 26 can bend without interfering with the channel defining inner peripheral surface portion 18. Further, a grip section 29 configured to grip a tissue and others is provided to the distal end treatment section 24. With an operation in the treatment instrument operation section 23, the grip section 29 is opened or closed and grips the tissue or the like.

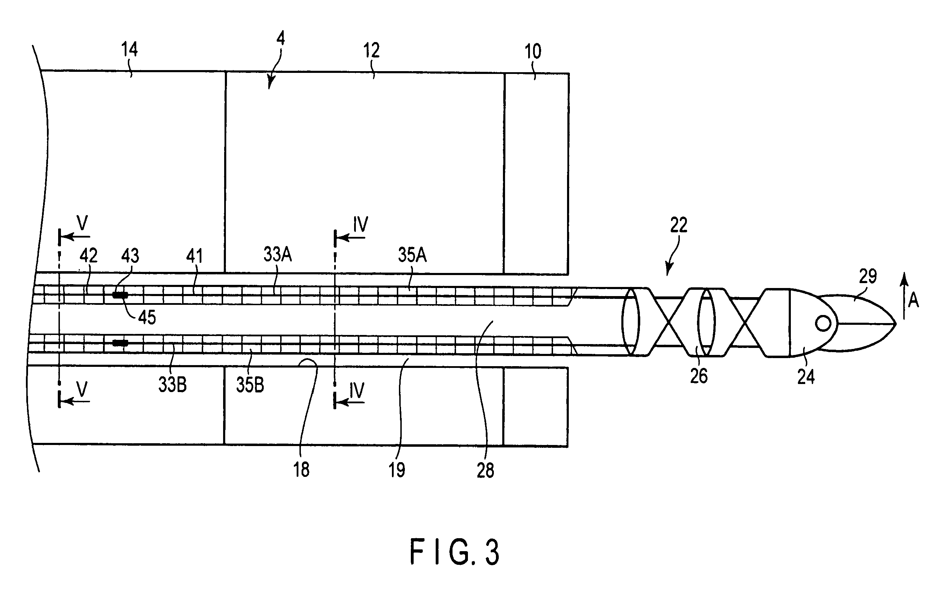

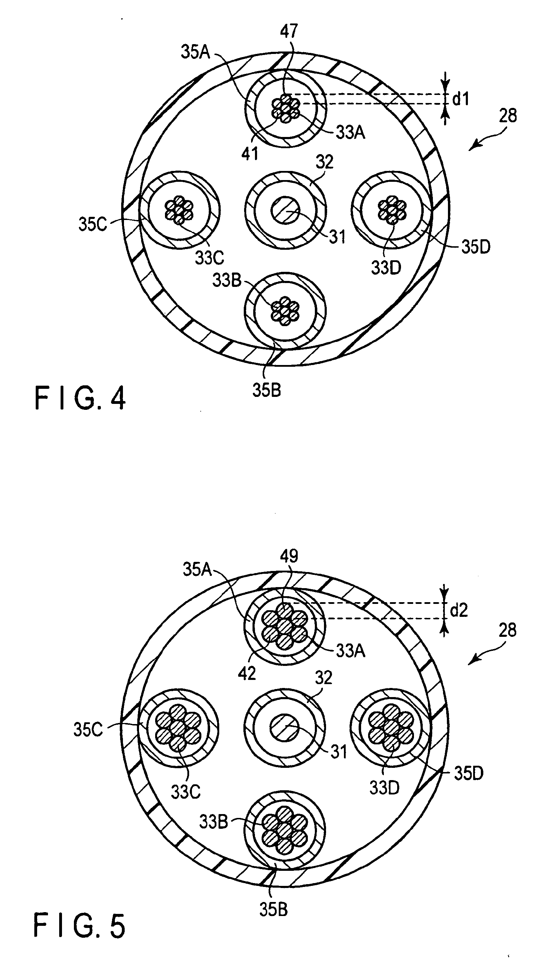

FIG. 3 is a view showing a part of the endoscopic device 1 on the distal direction side. FIG. 4 is a cross-sectional view taken along line IV-IV in FIG. 3, and FIG. 5 is a cross-sectional view taken along line V-V in FIG. 3. As shown in FIG. 4 and FIG. 5, a gripping operation wire 31 is extended in the treatment instrument insertion section 22 along the longitudinal directions. The gripping operation wire 31 is inserted into a gripping coil sheath 32. The gripping operation wire 31 has one end connected to the grip section 29, and the other end connected to the treatment instrument operation section 23. With an operation in the treatment instrument operation section 23, the gripping operation wire 31 moves forward or backward in the longitudinal directions. With the forward or backward movement of the gripping operation wire 31, the grip section 29 is opened or closed.

Moreover, two pairs of bending operation wires 33A to 33D are extended in the treatment instrument insertion section 22 along the longitudinal directions. The bending operation wires 33A to 33D are arranged to be approximately 90° apart from each other in circumferential directions of the treatment instrument insertion section 22. Each of the respective bending operation wires 33A to 33D is inserted in corresponding bending coil sheath 35A to 35D. For example, the bending operation wire 33A is inserted in the bending coil sheath 35A. Each of the bending operation wires 33A to 33D has one end connected to a distal end of the treatment instrument bending section 26, and the other end connected to the treatment instrument operation section 23. When the bending operation wires 33A to 33D are pulled or loosened based on an operation in the treatment instrument operation section 23, the treatment instrument bending section 26 bends. For example, in case of bending the treatment instrument bending section 26 in a direction of arrow A in FIG. 3, the bending operation wire 33A, which is one of the pair of bending operation wires 33A and 33B, is pulled, and the bending operation wire 33B, which is the other of the same, is loosened.

The bending operation wire 33A will now be described hereinafter. It is to be noted that the bending operation wires 33B to 33D have the same configuration as that of the bending operation wire 33A, and hence a description of these wires will be omitted. As shown in FIG. 3 to FIG. 5, the bending operation wire 33A includes a first wire portion 41, a second wire portion 42 provided to the proximal direction side of the first wire portion 41, and a wire joint portion 43 in which the first wire portion 41 and the second wire portion 42 are joined. In the wire joint portion 43, the first wire portion 41 and the second wire portion 42 are joined through a joint member 45. During a treatment at which the proximal end of the treatment instrument bending section 26 is placed to the distal direction side of the distal end of the endoscope insertion section 4, the wire joint portion 43 is placed to the proximal direction side of a proximal end of the endoscope bending section 12.

As shown in FIG. 4, the first wire portion 41 is formed by twisting seven first strands 47. Each first strand 47'has a first strand diameter d1. As shown in FIG. 5, the second wire portion 42 is formed by twisting seven second strands 49. That is, the second wire portion 42 is formed of the same number of the second strands 49 as the number of the first strands 47 forming the first wire portion 41. Each second strand 49 has a second strand diameter d2 larger than the first strand diameter d1. The number of the first strands 47 forming the first wire portion 41 and the number of the second strands 49 forming the second wire portion 42 are equalized, and the second strand diameter d2 of the second strand 49 is set larger than the first strand diameter d1 of the first strand 47, whereby the second wire portion 42 can have the higher rigidity than the first wire portion 41. That is, the bending operation wire 33A is a rigidity variable wire including the first wire portion 41, and the second wire portion 42 which is provided to the proximal direction side of the first wire portion 41 and has the higher rigidity than the first wire portion 41.

A function of the endoscopic device 1 not according to the present invention will now be described. When conducting observations through the endoscope 2 of the endoscopic device 1 and giving a treatment using the treatment instrument 20, the treatment instrument insertion section 22 is inserted into the treatment instrument insertion channel 19 from the treatment instrument insertion opening 16, and it is protruded from the distal end of the endoscope insertion section 4 (the distal end hard section 10) toward the distal direction side. At the time of a treatment using the treatment instrument 20, the proximal end of the treatment instrument bending section 26 is placed to the distal direction side of the distal end of the endoscope insertion section 4 (the distal end hard section 10). As a result, the treatment instrument bending section 26 can bend without interfering with the channel defining inner peripheral surface portion 18.

When the bending operation wires 33A to 33D are pulled or loosened based on an operation in the treatment instrument operation section 23, the treatment instrument bending section 26 bends. Each of the bending operation wires 33A to 33D includes the second wire portion 42 having the high rigidity. Therefore, when each of the bending operation wires 33A to 33D is pulled, the elongation of each of the pulled bending operation wires 33A to 33D is reduced.

Additionally, during a treatment at which the proximal end of the treatment instrument bending section 26 is placed to the distal direction side of the distal end of the endoscope insertion section 4 (the distal end hard section 10), the wire joint portion 43 is placed to the proximal direction side of the proximal end of the endoscope bending section 12. Therefore, the second wire portion 42 having the high rigidity is placed in the endoscope flexible tube section 14. That is, the first wire portion 41 having the low rigidity is placed in the endoscope bending section 12. Accordingly, the operability in the bending operation of the endoscope bending section 12 can be prevented from being decreased.

Therefore, the endoscopic device 1 having the above-described configuration can exercise the following effects. That is, in the endoscopic device 1, when the bending operation wires 33A to 33D are pulled or loosened based on an operation in the treatment instrument operation section 23, the treatment instrument bending section 26 bends. Each of the bending operation wires 33A to 33D includes the second wire portion 42 having the high rigidity. Therefore, when each of the bending operation wires 33A to 33D is pulled, the elongation of each of the pulled bending operation wires 33A to 33D is alleviated. When the elongation of each of the bending operation wires 33A to 33D is alleviated, a change in a bending amount of the treatment instrument bending section 26 with respect to a pulling amount of each of the bending operation wires 33A to 33D is reduced. As a result, the operability in the bending operation of the treatment instrument bending section 26 of the treatment instrument 20 can be improved.

Further, in a treatment at which the proximal end of the treatment instrument bending section 26 is placed to the distal direction side of the distal end of the endoscope insertion section 4 (the distal end hard section 10), the wire joint portion 43 is placed to the proximal direction side of the proximal end of the endoscope bending section 12. Therefore, the second wire portion 42 having the high rigidity is positioned in the endoscope flexible tube section 14. That is, the first wire portion 41 having the low rigidity is positioned in the endoscope bending section 12. Therefore, it is possible to avoid a reduction in operability in the bending operation of the endoscope bending section 12.

### (Modification of First Embodiment)

It is to be noted that, in the first embodiment, the first wire portion 41 of each of the bending operation wires 33A to 33D is formed of the seven first strands 47, and the second wire portion 42 of the same is formed of the seven second strands 49, but the present invention is not directed thereto. That is, any configuration can suffice as long as the number of the first strands 47 forming the first wire portion 41 is equal to the number of the second strands 49 forming the second wire portion 42 and the second strand diameter d2 of the second strand 49 is larger than the first strand diameter d1 of the first strand 47. As a result, the second wire portion 42 can have the rigidity higher than that of the first wire portion 41.

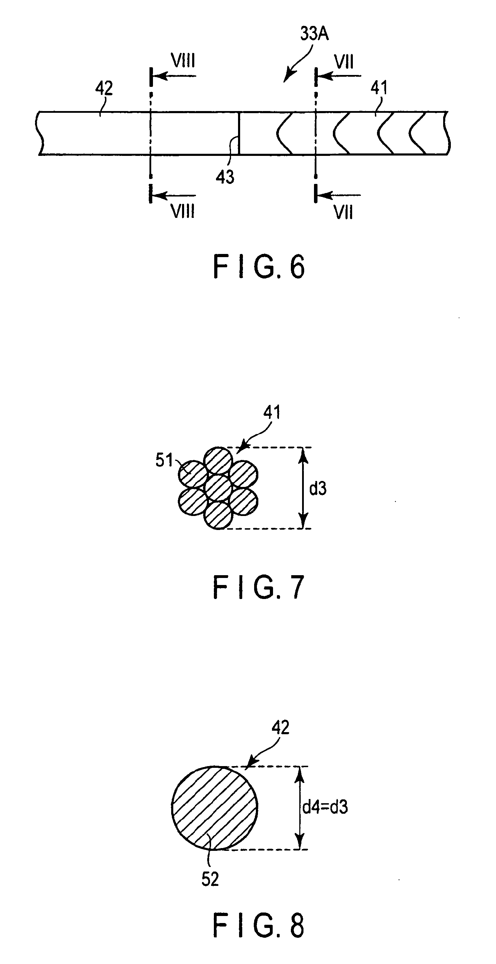

Furthermore, in the bending operation wires 33A to 33D, it is satisfactory to set the rigidity of the second wire portion 42 so that it can be higher than that of the first wire portion 41. FIG. 6 is a view showing a bending operation wire 33A according to a modification of the first embodiment. FIG. 7 is a cross-sectional view taken along line VII-VII in FIG. 6, and FIG. 8 is a cross-sectional view taken along line VIII-VIII in FIG. 6. As shown in FIG. 6 to FIG. 8, in this modification, a first wire portion 41 of the bending operation wire 33A is formed by twisting a plurality of (seven in this modification) first strands 51. The first wire portion 41 has a first wire diameter d3. A second wire portion 42 is formed of one second strand 52. The second wire portion 42 has a second wire diameter d4 equal to the first wire diameter d3. Here, the second wire diameter d4 is equal to a strand diameter of the second strand 52. As described above, in this modification, the first wire diameter d3 of the first wire portion 41 is set equal to the second wire diameter d4 of the second wire portion 42. Moreover, the first wire portion 41 is formed by twisting the first strands 41, whereas the second wire portion 42 is formed of the one second wire 52. As a result, the second wire portion 42 has the higher rigidity than the first wire portion 41.

Additionally, in this modification, at a wire joint portion 43 of the bending operation wire 33A, the first wire portion 41 is joined to the second wire portion 42 by welding. That is, a sufficient configuration is that the wire joint portion 43 where the first wire portion 41 and the second wire portion 42 are joined is provided. Further, at the time of a treatment in which a proximal end of a treatment instrument bending section 26 is placed to a distal direction side of a distal end of an endoscope insertion section 4 (a distal end hard section 10), it is satisfactory to place the wire joint portion 43 to a proximal direction side of a proximal end of an endoscope bending section 12.

Furthermore, in the first embodiment, the four bending operation wires 33A to 33D are the rigidity variable wires each including the first wire portion 41, and the second wire portion 42 which is provided to the proximal direction side of the first wire portion 41 and has the higher rigidity than the first wire portion 41, but the present invention is not restricted thereto. That is, it is possible to use the rigidity variable wire for at least one of the four bending operation wires 33A to 33D.

Moreover, in the first embodiment, the treatment instrument bending section 26 configured to bend in four directions, but the treatment instrument bending section 26 may be configured to bend in two directions. In this case, one pair of bending operation wires 33A and 33B are extended in the treatment instrument insertion section 22. Additionally, at least one of the pair of the bending operation wires 33A and 33B is the rigidity variable wire.

Further, in the first embodiment, the distal end treatment section 24 includes the grip section 29 configured to grip a tissue and the like, but the present invention is not directed thereto. For example, the distal end treatment section 24 may be a cautery knife configured to resect a diseased part. That is, it is satisfactory to provide the distal end treatment section 24 on the most distal direction side of the treatment instrument insertion section 22.

### (Second Embodiment)

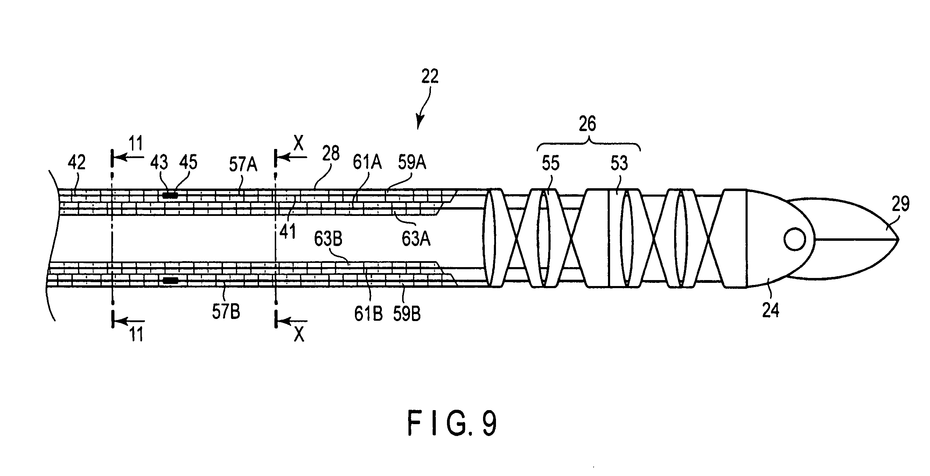

A second embodiment according to the present invention will now be described with reference to FIG. 9 to FIG. 11. It is to be noted that like reference numbers denote the same parts or parts having the same functions as those in the first embodiment to omit a description thereof.

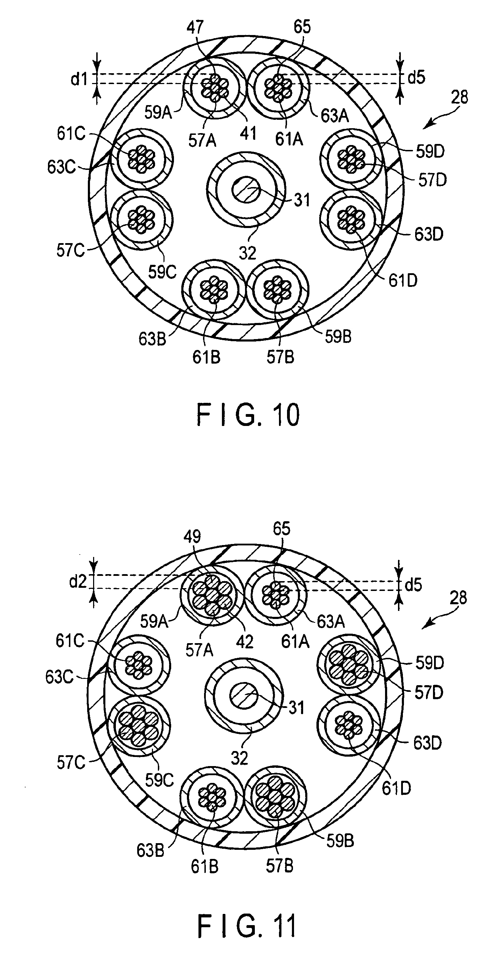

FIG. 9 is a view showing a part of a treatment instrument insertion section 22 on a distal direction side according to this embodiment. FIG. 10 is a cross-sectional view taken along line X-X in FIG. 9, and FIG. 11 is a cross-sectional view taken along line 11-11 in FIG. 9. As show in FIG. 9, like the first embodiment, a treatment instrument insertion section 22 includes a distal end treatment section 24, a treatment instrument bending section 26, and a treatment instrument tubular section 28. The treatment instrument bending section 26 includes a first bending section 53, and a second bending section 55 provided to a proximal direction side of the first bending section 53.

As shown in FIG. 9 to FIG. 11, in the treatment instrument insertion section 22, two pairs of first bending operation wires 57A to 57D are extended along longitudinal directions. The first bending operation wires 57A to 57D are arranged to be substantially 90° apart from each other in circumferential directions of the treatment instrument insertion section 22. Each of the first bending operation wires 57A to 57D is inserted through a corresponding first bending coil sheath 59A to 59D. For example, the first bending operation wire 57A is inserted through the first bending coil sheath 59A. Each of the first bending operation wires 57A to 57D has one end connected to a distal end of the first bending section 53 of the treatment instrument bending section 26, and the other end connected to the treatment instrument operation section 23. When the first bending operation wires 57A to 57D are pulled or loosened based on an operation in the treatment instrument operation section 23, the first bending section 53 bends.

Further, in the treatment instrument insertion section 22, two pairs of second bending operation wires 61A to 61D are extended in the longitudinal directions. The second bending operation wires 61A to 61D are arranged to be substantially 90° apart from each other in the circumferential directions of the treatment instrument insertion section 22. Furthermore, Each of the second bending operation wires 61A to 61D is arranged in substantially the same phases as the corresponding first bending operation wire 57A to 57D in the circumferential directions of the treatment instrument insertion section 22. For example, the second bending operation wire 61A is arranged in substantially the same phase as the first bending operation wire 57A in the circumferential directions of the treatment instrument insertion section 22. Each of the second bending operation wires 61A to 61D is inserted through a corresponding second bending coil sheath 63A to 63D. For example, the second bending operation wire 61A is inserted through the second bending coil sheath 63A. Each of the second bending operation wires 61A to 61D has one end connected to a distal end of the second bending section 55 of the treatment instrument bending section 26, and the other end connected to the treatment instrument operation section 23. When the second bending operation wires 61A to 61D are pulled or loosened based on an operation in the treatment instrument operation section 23, the second bending section 55 bends.

The first bending operation wire 57A will now be described hereinafter. It is to be noted that the first bending operation wires 57B to 57D have the same configuration as the first bending operation wire 57A, and hence a description of these wires 57B to 57D will be omitted. As shown in FIG. 9 to FIG. 11, the first bending operation wire 57A includes a first wire portion 41, a second wire portion 42 provided to the proximal direction side of the first wire portion 41, and a wire joint portion 43 in which the first wire portion 41 and the second wire portion 42 are joined. In the wire joint portion 43, the first wire portion 41 and the second wire portion 42 are joined through a joint member 45. At a treatment in which a proximal end of the treatment instrument bending section 26 is placed to the distal direction side of the distal end of the endoscope insertion section 4, the wire joint portion 43 is placed to the proximal direction side of a proximal end of the endoscope bending section 12.

As shown in FIG. 10, the first wire portion 41 is formed by twisting seven first strands 47. Each first strand 47 has a first strand diameter d1. As shown in FIG. 11, the second wire portion 42 is formed by twisting seven second strands 49. That is, the second wire portion 42 is formed of the same number of the second strands 49 as the number of the first strands 47 forming the first wire portion 41. Each second strand 49 has a second strand diameter d2 larger than the first strand diameter d1. When the number of the first strands 47 forming the first wire portion 41 is set equal to the number of the second strands 49 forming the second wire portion 42 and the second strand diameter d2 of the second strand 49 is set larger than the first strand diameter d1 of the first strand 47, the rigidity of the second wire portion 42 becomes higher than that of the first wire portion 41. That is, the first bending operation wire 57A is a rigidity variable wire including the first wire portion 41, and the second wire portion 42 which is provided to the proximal direction side of the first wire portion 41 and has the higher rigidity than the first wire portion 41.

The second bending operation wire 61A will now be described hereinafter. It is to be noted that the second bending operation wires 61B to 61D have the same configuration as that of the second bending operation wire 61A, and hence a description of these wires will be omitted.

The second bending operation wire 61A is a rigidity invariable wire whose rigidity is invariable from a distal end to a proximal end. The second bending operation wire 61A is formed by twisting seven third strand 65. That is, the second bending operation wire 61A is formed of the same number of the third strands 65 as the number of the second strands 49 forming the second wire portion 42 of each of the first bending operation wires 57A to 57D. Each third strand 65 has a third strand diameter d5 smaller than the second strand diameter d2. When the number of the third strands 65 forming the second bending operation wire 61A is set equal to the number of the second strands 49 forming the second wire portion 42 of each of the first bending operation wires 57A to 57D and the third strand diameter d5 of the third strand 65 is set smaller than the second strand diameter d2 of the second strand 49, the rigidity of the second bending operation wire 61A becomes smaller than that of the second wire portion 42.

A function of the endoscopic device 1 according to this embodiment will now be described. When conducting observations through an endoscope 2 of the endoscopic device 1 and giving a treatment using a treatment instrument 20, the treatment instrument insertion section 22 is inserted into a treatment instrument insertion channel 19 from a treatment instrument insertion opening 16 and protruded from the distal end of the endoscope insertion section 4 (the distal end hard section 10) toward the distal direction side. At the time of a treatment using the treatment instrument 20, the proximal end of the treatment instrument bending section 26 (a proximal end of the second bending section 55) is placed to the distal direction side of the distal end of the endoscope insertion section 4 (a distal end hard section 10). As a result, the treatment instrument bending section 26 can bend without interfering with a channel defining inner peripheral surface portion 18.

In the treatment instrument bending section 26, when the first bending operation wires 57A to 57D are pulled or loosened based on an operation in the treatment instrument operation section 23, the first bending section 53 bends. Each of the first bending operation wires 57A to 57D includes the second wire portion 42 having the high rigidity. Therefore, when each of the first bending operation wires 57A to 57D is pulled, the elongation of each of the pulled first bending operation wires 57A to 57D is reduced.

Further, when the second bending operation wires 61A to 61D are pulled or loosened based on an operation in the treatment instrument operation section 23, the second bending section 55 bends. Each of the second bending operation wires 61A to 61D has the rigidity that is invariable from the distal end to the proximal end, and this rigidity is smaller than that of the second wire portion 42 of each of the first bending operation wires 57A to 57D. Therefore, when the respective second bending operation wires 61A to 61D are pulled, the second bending operation wires 61A to 61D are apt to elongate as compared with the first bending operation wires 57A to 57D. However, at the time of an actual treatment using the treatment instrument 20, the first bending section 53 placed to the distal direction side must have the operability in the bending operation as compared with the second bending section 55. Therefore, if the operability in the bending operation of the first bending section 53 can be assured, the treatment in the treatment instrument 20 is not seriously affected.

Furthermore, At a treatment in which the proximal end of the treatment instrument bending section 26 (the proximal end of the second bending section 55) is placed to the distal direction side of the distal end of the endoscope insertion section 4 (the distal end hard section 10), the wire joint portion 43 of each of the first bending operation wires 57A to 57D is placed to the proximal direction side of the proximal end of the endoscope bending section 12. Therefore, in each of the first bending operation wires 57A to 57D, the second wire portion 42 having the high rigidity is placed in the endoscope flexible tube section 14, and the first wire portion 41 having the low rigidity is placed in the endoscope bending section 12. Moreover, the second bending operation wires 61A to 61D running through the endoscope bending section 12 have the low rigidity. Therefore, it is possible to prevent the operability in the bending operation of the endoscope bending section 12 from being reduced.

Additionally, the second bending operation wires 61A to 61D running through the endoscope flexible tube section 14 have the rigidity lower than that of the second wire portion 42 of each of the first bending operation wires 57A to 57D. Here, it is assumed that, like the first bending operation wires 57A to 57D, each of the second bending operation wires 61A to 61D is a rigidity variable wire including the first wire portion 41 and the second wire portion 42. In this case, the highly rigid second wire portions 42 of the eight bending operation wires 57A to 57D and 61A to 61D are placed in the endoscope flexible tube section 14. Therefore, the endoscope flexible tube section 14 is hardened, and its flexibility is deteriorated. Actually, since the endoscope flexible tube section 14 is configured to be looped or inverted in a body cavity, it is preferable for this section to have the flexibility to some extent. Therefore, in this embodiment, the rigidity of each of the second bending operation wires 61A to 61D is set invariable from the distal end to the proximal end, and the rigidity of each of the second bending operation wires 61A to 61D is set smaller than that of the second wire portion 42 of each of the first bending operation wires 57A to 57D. As a result, the flexibility of the endoscope flexible tube section 14 can be prevented from being deteriorated, and the operability of the endoscope 2 is assured.

Therefore, in the thus configured endoscopic device 1, in addition to the same effects as those of the first embodiment, the following effects can be exerted. That is, in the treatment instrument 20, the rigidity of each of the second bending operation wires 61A to 61D is set invariable from the distal end to the proximal end, and the rigidity of each of the second bending operation wires 61A to 61D is set smaller than that of the second wire portion 42 of each of the first bending operation wires 57A to 57D. Therefore, even when the two bending sections 53 and 55 are provided in the treatment instrument 20 and the number the bending operation wires 57A to 57D and 61A to 61D is increased, the flexibility of the endoscope flexible tube section 14 can be prevented from being deteriorated. As a result, the operability of the endoscope 2 can be assured.

### (Modification of Second Embodiment)

It is to be noted that, in the second embodiment, the number of the first strands 47 forming the first wire portion 41 of each of the first bending operation wires 57A to 57D is set equal to the number of the second strands 49 forming the second wire portion 42 and the second strand diameter d2 of the second wire 49 is set larger than the first strand diameter d1 of the first wire 47, but the present invention is not restricted thereto. For example, as described above, the first wire diameter d3 of the first wire portion 41 may be set equal to the second wire diameter d4 of the second wire portion 42, the first wire portion 41 may be formed by twisting the first strands 51, and the second wire portion 42 may be formed of one second strand 52. That is, in each of the first bending operation wires 57A to 57D, a satisfactory configuration is that the rigidity of the first wire portion 42 is higher than that of the second wire portion 42.

Additionally, in the second embodiment, each of the four second bending operation wires 61A to 61D is the rigidity invariable wire which has the rigidity invariable from the distal end to the proximal end and lower than that of the second wire portion 42 of each of the first bending operation wires 57A to 57D, but the present invention is not restricted thereto. That is, it is possible to adopt a configuration that at least one of the four second bending operation wires 61A to 61D is the rigidity invariable wire. For example, the second bending operation wires 61A to 61C may be the rigidity invariable wires, and the second bending operation wire 61D may be the rigidity variable wire including the first wire portion 41 and the second wire portion 42.

Further, in the second embodiment, although the second bending section 55 bends in four directions, the second bending section 55 may be configured to bend in two directions. In this case, a pair of second bending operation wires 61A and 61B are extended in the treatment instrument insertion section 22. Furthermore, at least one of the pair of second bending operation wires 61A and 61B is the rigidity invariable wire.

### (Third Embodiment)

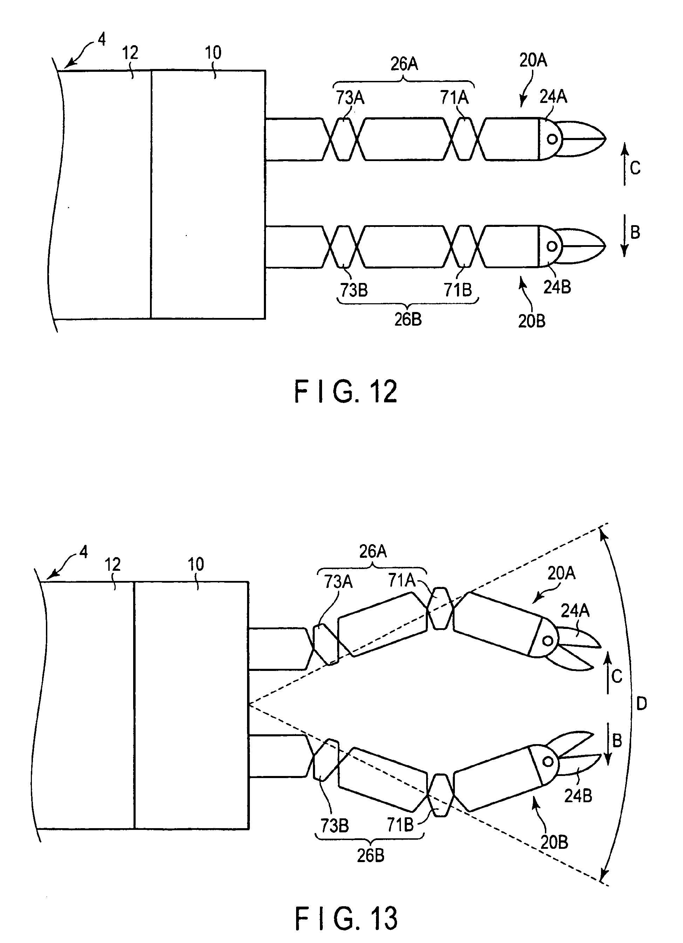

A third embodiment according to the present invention will now be described with reference to FIG. 12 to FIG. 16. It is to be noted that like reference numbers denote the same parts or parts having the same functions as those in the first embodiment to omit a description thereof.

FIG. 12 is a view showing a part of an endoscopic device 1 on a distal direction side according to this embodiment. As shown in FIG. 12, in the endoscopic device 1, a first treatment instrument 20A and a second treatment instrument 20B are inserted through an endoscope 2 (in the following description, each structure concerning the first treatment instrument 20A is denoted by reference number having a postfix A, and each structure concerning the second treatment instrument 20B is denoted by reference number having a postfix B). A treatment instrument bending section 26A of the first treatment instrument 20A includes a first bending section 71A, and a second bending section 73A provided to a proximal direction side of the first bending section 71A. The first bending section 71A and the second bending section 73A bend in two directions, i.e., the direction of arrow B and the direction of arrow C in FIG. 12. Furthermore, a treatment instrument bending section 26B of the second treatment instrument 20B includes a first bending section 71B and a second bending section 73B provided to the proximal direction side of the first bending section 71A. The first bending section 71B and the second bending section 73B bend in two directions, i.e., the direction of arrow B and the direction of arrow C in FIG. 12.

FIG. 13 is a view showing the part of the endoscopic device 1 on the distal direction side at the time of a treatment using the first treatment instrument 20A and the second treatment instrument 20B. As shown in FIG. 13, at the time of a treatment using the first treatment instrument 20A and the second treatment instrument 20B, a distal end treatment section 24A of the first treatment instrument 20A and a distal end treatment section 24B of the second treatment instrument 20B are placed in an observation range D of the endoscope 2. Therefore, in the first treatment instrument 20A, the first bending section 71A is bent in the direction of arrow B in FIG. 13 with a high frequency, and the second bending section 73A is bent in the direction of arrow C in FIG. 13 with a high frequency. That is, the direction of arrow B is a high-frequency bending direction in which the first bending section 71A is bent with the high frequency in the first bending section 71A, and the direction of arrow C is a high-frequency bending direction in which the second bending section 73A is bent with the high frequency in the second bending section 73A. Furthermore, in the second treatment instrument 20B, the first bending section 71B is bent in the direction of arrow C in FIG. 13 with the high frequency, and the second bending section 73B is bent in the direction of arrow B with the high frequency. That is, the direction of arrow C is the high-frequency bending direction in which the first bending section 71B is bent with the high frequency in the first bending section 71B, and the direction of arrow B is the high-frequency bending direction in which the second bending section 73B is bent with the high frequency in the second bending portion 73B.

A configuration that bends the first bending section 71A and the second bending section 73A in the first treatment instrument 20A will now be described. It is to be noted that a configuration that bends the first bending section 71B and the second bending section 73B in the second treatment instrument 20B is the same as the configuration that bends the first bending section 71A and the second bending section 73A, thereby omitting a description thereof.

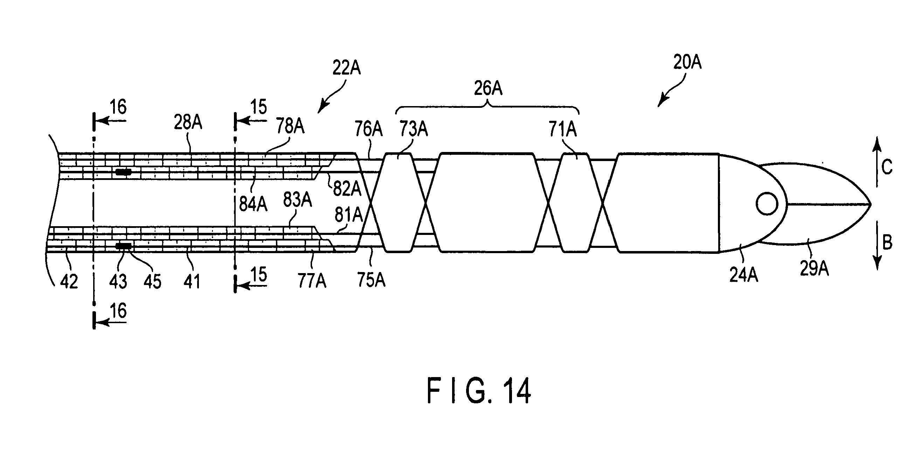

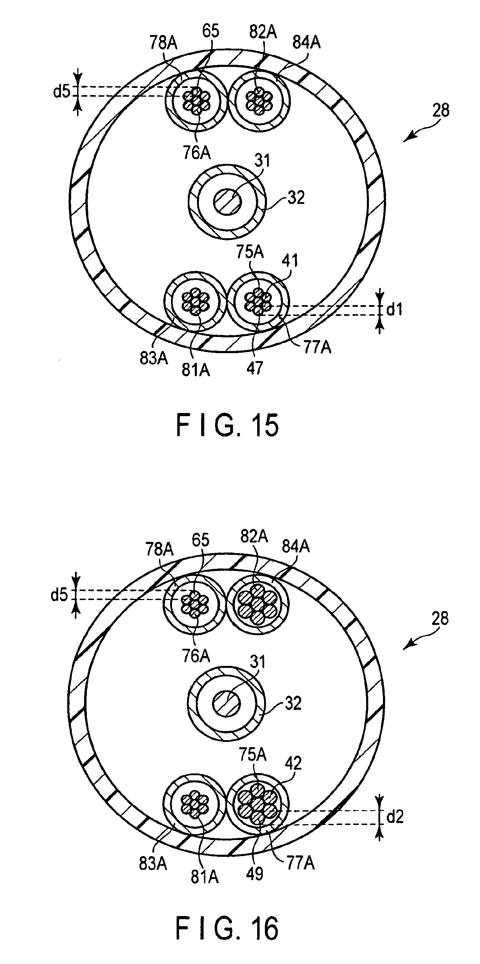

FIG. 14 is a view showing a part of the first treatment instrument 20A on the distal direction side. FIG. 15 is a cross-sectional view taken along line 15-15 in FIG. 14, and FIG. 16 is a cross-sectional view taken along line 16-16 in FIG. 14. As shown in FIG. 14 to FIG. 16, in the treatment instrument insertion section 22A, a pair of first bending operation wires 75A and 76A are extended in longitudinal directions. The first bending operation wires 75A and 76A are arranged to be substantially 180° apart from each other in circumferential directions of the treatment instrument insertion section 22A. Each of the first bending operation wires 75A and 76A is inserted through a corresponding first bending coil sheath 77A and 78A. For example, the first bending operation wire 75A is inserted through the first bending coil sheath 77A. Each of the first bending operation wires 75A and 76A has one end connected to a distal end of the first bending section 71A of the treatment instrument bending section 26A, and the other end connected to a treatment instrument operation section 23. When the first bending operation wires 75A and 76A are pulled or loosened based on an operation in the treatment instrument operation section 23, the first bending section 71A bends.

Here, when the first bending section 71A bends in the high-frequency bending direction of the first bending section 71A which is the direction of arrow B in FIG. 14, the first bending operation wire 75A is pulled, and the first bending operation wire 76A is loosened. That is, the first bending operation wire 75A is a high-frequency pulling wire configured to be pulled when the first bending section 71A is bent in the high-frequency bending direction. Moreover, the first bending operation wire 76A is a high-frequency loosening wire configured to be loosened when the first bending section 71A is bent in the high-frequency bending direction.

Additionally, in the treatment instrument insertion section 22A, a pair of second bending operation wires 81A and 82A are extended in the longitudinal directions. The second bending operation wires 81A and 82A are arranged to be substantially 180° apart from each other in the circumferential directions of the treatment instrument insertion section 22A. Further, each of the respective second bending operation wires 81A and 82A is arranged in substantially the same phases as the corresponding first bending operation wire 75A and 76A in the circumferential directions of the treatment instrument insertion section 22A. For example, the second bending operation wire 81A is arranged in substantially the same phase as the first bending operation wire 75A in the circumferential directions of the treatment instrument insertion section 22A. Each of the second bending operation wires 81A and 82A is inserted through a corresponding second bending coil sheath 83A and 84A. For example, the second bending operation wire 81A is inserted through the second bending coil sheath 83A. Each of the second bending operation wires 81A and 82A has one end connected to a distal end of the second bending section 73A of the treatment instrument bending section 26A, and the other end connected to the treatment instrument operation section 23. When the second bending operation wires 81A and 82A are pulled or loosened based on an operation in the treatment instrument operation section 23, the second bending section 73A bends.

Here, when the second bending section 73A is bent in the high-frequency bending direction of the second bending section 73A which is the direction of arrow C in FIG. 14, the second bending operation wire 81A is loosened, and the second bending operation wire 82A is pulled. That is, the second bending operation wire 81A is a high-frequency loosening wire configured to be loosened when the second bending section 73A is bent in the high-frequency bending direction. Additionally, the second bending operation wire 82A is a high-frequency pulling wire configured to be pulled when the second bending section 73A is bent in the high-frequency bending direction.

The first bending operation wire 75A which is the high-frequency pulling wire will now be described. It is to be noted that the second bending operation wire 82A as the high-frequency pulling wire has the same configuration as the first bending operation wire 75A, thereby omitting a description thereof. As shown in FIG. 14 to FIG. 16, the first bending operation wire 75A includes a first wire portion 41, a second wire portion 42 which is provided to the proximal direction side of the first wire portion 41, and a wire joint portion 43 in which the first wire portion 41 and the second wire portion 42 are joined. In the wire joint portion 43, the first wire portion 41 and the second wire portion 42 are joined through a joint member 45. At a treatment in which a proximal end of the treatment instrument bending section 26A is placed to the distal direction side of a distal end of the endoscope insertion section 4, the wire joint portion 43 is placed to the proximal direction side of a proximal end of the endoscope bending section 12.

As shown in FIG. 15, the first wire portion 41 is formed by twisting seven first strands 47. Each first strand 47 has a first strand diameter d1. As shown in FIG. 16, the second wire portion 42 is formed by twisting seven second strand 49. That is, the second wire portion 42 is formed of the same number of the second strands 49 as the number of the first strands 47 forming the first wire portion 41. Each second strand 49 has a second strand diameter d2 larger than the first strand diameter d1. When the number of the first strands 47 forming the first wire portion 41 is set equal to the number of the second strands 49 forming the second wire portion 42 and the second strand diameter d2 is set larger than the first strand diameter d1 of the first strand 47, the rigidity of the second wire portion 42 becomes higher than that of the first wire portion 41. That is, the first bending operation wire 75A is a rigidity variable wire including the first wire portion 41, and the second wire portion 42 which is provided to the proximal direction side of the first wire portion 41 and has the rigidity higher than that of the first wire portion 41.

The first bending operation wire 76A as the high-frequency loosening wire will now be described. It is to be noted that the second bending operation wire 81A as the high-frequency loosening wire has the same configuration as the first bending operation wire 76A, thereby omitting a description thereof.

The first bending operation wire 76A is a rigidity invariable wire whose rigidity is invariable from a distal end to a proximal end. The first bending operation wire 76A is formed by twisting seven third strands 65. That is, the first bending operation wire 76A is formed of the same number of the third strands 65 as the number of the second strands 49 forming the second wire portion 42 of each high-frequency pulling wire (75A, 82A). Each third strand 65 has a third strand diameter d5 smaller than the second strand diameter d2. When the number of the third strands 65 forming the first bending operation wire 76A is set equal to the number of the second strands 49 forming the second wire portion 42 of each high-frequency pulling wire (75A, 82A) and the third strand diameter d5 of the third strand 65 is set smaller than the second strand diameter d2 of the second strand 49, the rigidity of the first bending operation wire 76A becomes smaller than that of the second wire portion 42.

A function of the endoscopic device 1 according to this embodiment will now be described. When conducting observations through an endoscope 2 of the endoscopic device 1 and giving a treatment using the first treatment instrument 20A and the second treatment instrument 20B, the treatment instrument insertion section 22A of the first treatment instrument 20A is inserted into a treatment instrument insertion channel 19A from a treatment instrument insertion opening 16 and protruded from the distal end of an endoscope insertion section 4 (a distal end hard section 10) to the distal direction side. At the time of a treatment using the first treatment instrument 20A, the proximal end of the treatment instrument bending section 26A (a proximal end of the second bending section 73A) is placed to the distal direction side of the distal end of the endoscope insertion section 4 (the distal end hard section 10). As a result, the treatment instrument bending section 26A can bend without interfering with the a channel defining inner peripheral surface portion 18A. In regard to the second treatment instrument 20B, like the first treatment instrument 20A, a treatment is given.

In the treatment instrument bending section 26A of the first treatment instrument 20A, when the first bending operation wires 75A and 76A are pulled or loosened based on an operation in the treatment instrument operation section 23, the first bending section 71A bends. In the first bending section 71A, the direction of arrow B in FIG. 14 is the high-frequency bending direction in which the first bending section 71A is bent with a high frequency. When the first bending section 71A is bent in the high-frequency bending direction of the first bending section 71A, the first bending operation wire 75A is pulled, and the first bending operation wire 76A is loosened. That is, the first bending operation wire 75A is the high-frequency pulling wire, and the first bending operation wire 76A is the high-frequency loosening wire.

Further, when the second bending operation wires 81A and 82B are pulled or loosened based on an operation in the treatment instrument operation section 23, the second bending section 73A bends. In the second bending section 73A, the direction of arrow C in FIG. 14 is a high-frequency bending direction in which the second bending section 73A is bent with a high frequency. When the second bending section 73A is bent in the high-frequency bending direction of the second bending section 73A, the second bending operation wire 81A is loosened, and the second bending operation wire 82A is pulled. That is, the second bending operation wire 81A serves as the high-frequency loosening wire, and the second bending operation wire 82A serves as the high-frequency pulling wire.

Each of the high-frequency pulling wires (75A, 82A) includes the second wire portion 42 having the high rigidity. Therefore, when each of the high-frequency pulling wires (75A and 82A) is pulled, the elongation of the pulled high-frequency pulling wire is reduced. Furthermore, each of the high-frequency loosening wires (76A, 81A) has the uniform rigidity from the distal end to the proximal end, and this rigidity is smaller than that of the second wire portion 42 of the high-frequency pulling wire (75A, 82A). Therefore, when each of the high-frequency loosening wires (76A, 81A) is pulled, the high-frequency loosening wire is apt to elongate as compared with the high-frequency pulling wire (75A, 82A). However, at the time of an actual treatment using the first treatment instrument 20A, each of the first bending section 71A and the second bending section 73A is bent in the high-frequency bending direction with a high frequency. Therefore, a frequency that each high-frequency loosening wire (76A, 81A) is pulled at the time of the treatment is low. Accordingly, even if each high-frequency loosening wire (76A, 81A) is configured to easily elongate, the treatment using the first treatment instrument 20A is not greatly affected. It is to be noted that, in regard to the second treatment instrument 20B, like the treatment instrument bending section 26A of the first treatment instrument 20A, the treatment instrument bending section 26B bends.

Moreover, at a treatment in which the proximal end of the treatment instrument bending section 26A (the proximal end of the second bending section 73A) of the first treatment instrument 20A is placed to the distal direction side of the distal end of the endoscope insertion section 4 (the distal end hard section 10), the wire joint portion 43 of each high-frequency pulling wire (75A, 82A) is placed to the proximal direction side of the proximal end of the endoscope bending section 12. Therefore, in the high-frequency pulling wire (75A, 82A), the second wire portion 42 having the high rigidity is placed in the endoscope flexible tube section 14, and the first wire portion 41 having the low rigidity is placed in the endoscope bending section 12. Additionally, each high-frequency loosening wire (76A, 81A) running through the endoscope bending section 12 has the low rigidity. The bending operation wires (75B, 76B, 81B, 82B) provided in the second treatment instrument 20B have the same configuration as the bending operation wires (75A, 76A, 81A, 82A) in the first treatment instrument 20A. Therefore, it is possible to prevent the operability in the bending operation of the endoscope bending section 12 from being deteriorated.

Additionally, each high-frequency loosening wire (76A, 81A) of the first treatment instrument 20A running through the endoscope flexible tube section 14 has the rigidity lower than that of the second wire portion 42 of each high-frequency pulling wire (75A, 82A). Here, it is assumed that, like the high-frequency pulling wire (75A, 82A), the high-frequency loosening wire (76A, 81A) is the rigidity variable wire including the first wire portion 41 and the second wire portion 42. In this case, the highly rigid second wire portions 42 of the bending operation wires 75A, 76A, 81A, and 82A are placed in the endoscope flexible tube section 14. Therefore, the endoscope flexible tube section 14 is hardened, and its flexibility is deteriorated. Since the endoscope flexible tube section 14 is configured to be actually looped or inverted in a body cavity, it is preferable for the endoscope flexible tube section 14 to have the flexibility to some extent. Therefore, in this embodiment, each high-frequency loosening wire (76A, 81A) has the rigidity that is invariable from the distal end to the proximal end, and the rigidity of the high-frequency loosening wire is set smaller than that of the second wire portion 42 of the high-frequency pulling wire (75A, 82A). The bending operation wires (75B, 76B, 81B, 82B) provided in the second treatment instrument 20B have the same configurations as those of the bending operation wires (75A, 76A, 81A, 82A) in the first treatment instrument 20A. Therefore, the flexibility of the endoscope flexible tube section 14 is prevented from being lowered, and the operability of the endoscope 2 is assured.

Therefore, in the thus configured endoscopic device 1, in addition to the same effects as those of the first embodiment, the following effects are exerted. That is, in the first treatment instrument 20A and the second treatment instrument 20B, each high-frequency loosening wire (76A, 76B, 81A, 81B) has the rigidity that is invariable from the distal end of the proximal end, and the rigidity of the high-frequency loosening wire is set smaller than that of the second wire portion 42 of the high-frequency pulling wire (75A, 75B, 82A, 82B). Therefore, even when the two treatment instruments 20A and 20B are provided and the number of the bending operation wires (75A, 75B, 76A, 76B, 81A, 81B, 82A, 82B) is increased, the flexibility of the endoscope flexible tube section 14 can be prevented from being lowered. As a result, the operability of the endoscope 2 can be assured.

### (Modification of Third Embodiment)

It is to be noted that the number of the first strands 47 forming the first wire portion 41 of each high-frequency pulling wire (75A, 75B, 82A, 82B) is set equal to the number of the second strands 49 forming the second wire portion 42, and the second strand diameter d2 of each second strand 49 is set larger than the first strand diameter d1 of each first strand 47, but the present invention is not restricted thereto. For example, as described above, the first wire diameter d3 of each first wire portion 41 may be set equal to the second wire diameter d4 of each second wire portion 42, the first wire portion 41 may be formed by twisting the first strands 51, and the second wire portion 42 may be formed of the single second strand 52. That is, in each high-frequency pulling wire (75A, 75B, 82A, 82B), setting the rigidity of the second wire portion 42 higher than that of the first wire portion 41 can suffice.

Furthermore, in the third embodiment, the first bending section 71A and the second bending section 73A in the first treatment instrument 20A bend in the two directions, and the first bending section 71B and the second bending section 73B in the second treatment instrument 20B bend in the two directions, but the present invention is not restricted thereto. For example, the first bending section 71A may bend in four directions. In this case, four first bending operation wires configured to bend the first bending section 71A are provided. Moreover, each first bending operation wire includes a high-frequency pulling wire configured to be pulled when the first bending section 71A is bent in the high-frequency bending direction, and a high-frequency loosening wire configured to be loosened when the first bending section 71A is bent in the high-frequency bending direction. Additionally, the high-frequency pulling wire serves as the rigidity variable wire, and the high-frequency loosening wire serves as the rigidity invariable wire.

Further, in the third embodiment, the two bending sections 71A and 73A are provided in the first treatment instrument 20A, but the present invention is not restricted thereto. For example, only one bending section may be provided in the first treatment instrument 20A. In this case, two bending operation wires configured to bend the bending section are provided. Furthermore, one of the two bending operation wires serves as a high-frequency pulling wire configured to be pulled when the bending section is bent in the high-frequency bending direction, and the other of the two bending operation wires serves as a high-frequency loosening wire configured to be loosened when the bending section is bent in the high-frequency bending direction. Moreover, the high-frequency pulling wire functions as the rigidity variable wire, and the high-frequency loosening wire functions as the rigidity invariable wire.

Additionally, in the third embodiment, although the two treatment instruments 20A and 20B are provided, the present invention is not restricted thereto. For example, the treatment instrument 20A alone may be inserted into the endoscope 2.

That is, it is sufficient for the bending operation wire to include the high-frequency pulling wire (75A or 82A in the third embodiment) configured to be pulled when the treatment instrument bending section 26 is bent in the high-frequency bending direction as a direction in which the treatment instrument bending unit 26 is bent with a high frequency, and the high-frequency loosening wire (76A or 81A in the third embodiment) configured to be loosened when the treatment instrument bending section 26 is bent in the high-frequency bending direction. Further, the configuration is satisfactory when the high-frequency pulling wire consists of the rigidity variable wire and the high-frequency loosening wire consists of the rigidity invariable wire.

Incidentally, the present invention is not limited to the above embodiments and various modifications can naturally be made without deviating from the scope of the present invention, as claimed.

## Claims

1. An endoscopic device (1) comprising:
an endoscope insertion section (4) which includes an endoscope bending section (12) configured to bend, and an endoscope flexible tube section (14) provided to a proximal direction side of the endoscope bending section (12), and which is configured to be inserted into a body cavity;
a treatment instrument insertion section (22) which includes a treatment instrument bending section (26) configured to bend, and which is configured to be inserted into the body cavity through a treatment instrument insertion channel (19) extended in the endoscope insertion section (4) along longitudinal directions;
a treatment instrument operation section (23) which is provided to the proximal direction side of the treatment instrument insertion section (22); and
a bending operation wire (57A-57D,61A-61D) which has one end connected to the treatment instrument bending section (26), and the other end connected to the treatment instrument operation section (23), and which is configured to bend the treatment instrument bending section (26) when pulled or loosened by an operation in the treatment instrument operation section (23),
wherein the bending operation wire (57A-57D,61A-61D) includes a rigidity variable wire (57A-57D) which includes a first wire portion (41), a second wire portion (42) provided to the proximal direction side of the first wire portion (41) and having a rigidity higher than that of the first wire portion (41), and a wire joint portion (43) in which the first wire portion (41) and the second wire portion (42) are joined, the wire joint portion (43) being configured to be placed to the proximal direction side of a proximal end of the endoscope bending section (12) at a treatment in which a proximal end of the treatment instrument bending section (26) is placed to a distal direction side of a distal end of the endoscope insertion section (4),
the treatment instrument bending section (26) includes a first bending section (53), and a second bending section (55) provided to the proximal direction side of the first bending section (53),
the bending operation wire (57A-57D,61A-61D) includes a first bending operation wire (57A-57D) configured to bend the first bending section (53) when pulled or loosened, and a second bending operation wire (61A-61D) configured to bend the second bending section (55) when pulled or loosened,
the first bending operation wire (57A-57D) consists of the rigidity variable wire (57A-57D), and
the second bending operation wire (61A-61D) includes a rigidity invariable wire (61A-61D) a rigidity of which is invariable from a distal end to a proximal end and is smaller than the rigidity of the second wire portion (42) of the rigidity variable wire (57A-57D).

2. The endoscopic device (1) according to claim 1,
**characterized in that** the first wire portion (41) is formed by twisting first strands (47) each of which has a first strand diameter (d1), and
the second wire portion (42) is formed by twisting second strands (49) each of which has a second strand diameter (d2) larger than the first strand diameter (d1) of each of the first strands (47).

3. The endoscopic device (1) according to claim 2,
**characterized in that** the second wire portion (42) is formed of the same number of the second strands (49) as the number of the first strands (47) forming the first wire portion (41).

4. The endoscopic device (1) according to claim 1,
**characterized in that** the first wire portion (41) is formed by twisting first strands (51) and has a first wire diameter (d3), and
the second wire portion (42) is formed of only one second strand (52) and has a second wire diameter (d4) equal to the first wire diameter (d3).

## Patentansprüche

1. Endoskopvorrichtung (1) mit:
einem Endoskop-Einführbereich (4), der einen zum Biegen ausgebildeten Endoskop-Biegebereich (12) und einen flexiblen, auf der proximalen Richtungsseite des Endoskop-Biegebereichs (12) vorgesehenen Endoskop-Rohrbereich (14) aufweist und der zum Einführen in einen Köperhohlraum ausgebildet ist;
einem Behandlungsinstrument-Einführbereich (22), der einen zum Biegen ausgebildeten Behandlungsinstrument-Biegebereich (26) aufweist und der dazu ausgebildet ist, durch einen sich in Längsrichtungen in dem Endoskop-Einführbereich (4) erstreckenden Behandlungsinstrument-Einführkanal (19) in den Köperhohlraum eingeführt zu werden;
einem Behandlungsinstrument-Betätigungsbereich (23), der auf der proximalen Richtungsseite des Behandlungsinstrument-Einführbereichs (22) vorgesehen ist; und mit
einem Biegebetätigungsdraht (57A-57D, 61A-61D) dessen eines Ende mit dem Behandlungsinstrument-Biegebereich (26) und dessen anderes Ende mit dem Behandlungsinstrument-Betätigungsbereich (23) verbunden ist und der dazu ausgebildet ist, den Behandlungsinstrument-Biegebereich (26) zu biegen, wenn er durch eine Betätigung in dem Behandlungsinstrument-Betätigungsbereich (23) angezogen oder gelockert wird,
wobei der Biegebetätigungsdraht (57A-57D, 61A-61D) einen Draht (57A-57D) mit variabler Biegesteifheit aufweist, der einen ersten Drahtbereich (41), einen zweiten Drahtbereich (42), der auf der proximalen Richtungsseite des ersten Drahtbereichs (41) vorgesehen und dessen Biegesteifheit höher als die des ersten Drahtbereichs (41) ist, und ein Drahtverbindungsstück (43), in dem der erste Drahtbereich (41) und der zweite Drahtbereich (42) verbunden sind, aufweist, wobei das Drahtverbindungsstück (43) dazu ausgebildet ist, bei einer Behandlung, bei der ein proximales Ende des Behandlungsinstrument-Biegebereichs (26) an einer distalen Richtungsseite eines distalen Endes des Endoskop-Einführbereichs (4) angesetzt wird, an der proximalen Richtungsseite eines proximalen Endes des Endoskop-Biegebereichs (12) angesetzt zu werden,
der Behandlungsinstrument-Biegebereich (26) einen ersten Biegebereich (53) und einen zweiten Biegebereich (55) aufweist, der auf der proximalen Richtungsseite des ersten Biegebereichs (53) vorgesehen ist,
der Biegebetätigungsdraht (57A-57D, 61A-61D) einen ersten Biegebetätigungsdraht (57A-57D), der dazu ausgebildet ist, den ersten Biegebereich (53) zu biegen, wenn er angezogen oder gelockert wird, und einen zweiten Biegebetätigungsdraht (61A-61D) aufweist, der dazu ausgebildet ist, den zweiten Biegebereich (55) zu biegen, wenn er angezogen oder gelockert wird,
der erste Biegebetätigungsdraht (57A-57D) aus dem Draht (57A-57D) mit variabler Biegesteifheit besteht, und
der zweite Biegebetätigungsdraht (61A-61D) einen Draht (61A-61D) mit unveränderlicher Biegesteifheit aufweist, dessen Biegesteifheit von einem distalen Ende zu einem proximalen Ende unveränderlich ist und geringer ist als die Biegesteifheit des zweiten Drahtbereichs (42) des Drahts (57A-57D) mit variabler Biegesteifheit.

2. Endoskopvorrichtung (1) nach Anspruch 1,
**dadurch gekennzeichnet, dass** der erste Drahtbereich (41) durch Verdrillen erster Einzeldrähte (47) gebildet ist, von denen jeder einen ersten Einzeldrahtdurchmesser (d1) aufweist, und
der zweite Drahtbereich (42)) durch Verdrillen zweiter Einzeldrähte (49) gebildet ist, von denen jeder einen zweiten Einzeldrahtdurchmesser (d2) aufweist, der größer als der erst Einzeldrahtdurchmesser (d1) jedes der ersten erster Einzeldrähte (47) ist.

3. Endoskopvorrichtung (1) nach Anspruch 2,
**dadurch gekennzeichnet, dass** der zweite Drahtbereich (42) aus einer Anzahl von zweiten Einzeldrähten (49) gebildet ist, die der Anzahl der den ersten Drahtbereich (41) bildenden ersten Einzeldrähte (47) entspricht.

4. Endoskopvorrichtung (1) nach Anspruch 1,
**dadurch gekennzeichnet, dass** der erste Drahtbereich (41) durch Verdrillen erster Einzeldrähte (51) gebildet ist und einen ersten Drahtdurchmesser (d3) aufweist, und
dass der zweite Drahtbereich (42) aus nur einem zweiten Einzeldraht (52) gebildet ist und einen dem ersten Drahtdurchmesser (d3) entsprechenden zweiten Drahtdurchmesser (d4) aufweist.

## Revendications

1. Dispositif d'endoscope (1) comprenant :
une section d'insertion d'endoscope (4) qui comprend une section de courbure d'endoscope (12) configurée pour se courber, et une section de tube flexible d'endoscope (14) prévue sur un côté dans la direction proximale de la section de courbure d'endoscope (12), et qui est configurée pour être insérée dans une cavité de corps ;
une section d'insertion d'instrument de traitement (22) qui comprend une section de courbure d'instrument de traitement (26) configurée pour se courber, et qui est configurée pour être insérée dans la cavité de corps à travers un canal d'insertion d'instrument de traitement (19) s'étendant dans la section d'insertion d'endoscope (4) le long des directions longitudinales ;
une section d'actionnement d'instrument de traitement (23) qui est prévue sur le côté dans la direction proximale de la section d'insertion d'instrument de traitement (22) ; et
un câble d'actionnement de courbure (57A à 57D, 61A à 61D) qui comporte une extrémité connectée à la section de courbure d'instrument de traitement (26), et l'autre extrémité connectée à la section d'actionnement d'instrument de traitement (23), et qui est configuré pour courber la section de courbure d'instrument de traitement (26) lorsqu'il est tiré ou relâché par une opération dans la section d'actionnement d'instrument de traitement (23),
dans lequel le câble d'actionnement de courbure (57A à 57D, 61A à 61D) comprend un câble à rigidité variable (57A à 57D) qui comprend une première partie de câble (41), une deuxième partie de câble (42) prévue sur le côté de la direction proximale de la première partie de câble (41) et présentant une rigidité supérieure à celle de la première partie de câble (41), et une partie de raccord de câbles (43) dans laquelle la première partie de câble (41) et la deuxième partie de câble (42) sont raccordées, la partie de raccord de câble (43) étant configurée pour être placée sur le côté dans la direction proximale d'une extrémité proximale de la section de courbure d'endoscope (12) au niveau d'une partie dans laquelle une extrémité proximale de la section de courbure d'instrument de traitement (26) est placée sur un côté dans la direction distale d'une extrémité distale de la section d'insertion d'endoscope (4),
la section de courbure d'instrument de traitement (26) comprend une première section de courbure (53), et une deuxième section de courbure (55) prévue sur le côté dans la direction proximale de la première section de courbure (53),
le câble d'actionnement de courbure (57A à 57D, 61A à 61D) comprend un premier câble d'actionnement de courbure (57A à 57D) configuré pour courber la première section de courbure (53) lorsqu'il est tiré ou relâché, et un deuxième câble d'actionnement de courbure (61A à 61D) configuré pour courber la deuxième section de courbure (55) lorsqu'il est tiré ou relâché,
le premier câble d'actionnement de courbure (57A à 57D) est composé du câble à rigidité variable (57A à 57D), et
le deuxième câble d'actionnement de courbure (61A à 61D) comprend un câble à rigidité invariable (61A à 61D) dont une rigidité ne varie pas d'une extrémité distale à une extrémité proximale et est inférieure à la rigidité de la deuxième partie de câble (42) du câble à rigidité variable (57A à 57D).

2. Dispositif d'endoscope (1) selon la revendication 1,
**caractérisé en ce que** la première partie de câble (41) est formée en enroulant des premiers torons (47) dont chacun présente un premier diamètre de toron (d1), et
la deuxième partie de câble (42) est formée en enroulant des deuxièmes torons (49) dont chacun présente un deuxième diamètre de toron (d2) supérieur au premier diamètre de toron (d1) de chacun des premiers torons (47).

3. Dispositif d'endoscope (1) selon la revendication 2,
**caractérisé en ce que** la deuxième partie de câble (42) est formée du même nombre de deuxièmes torons (49) que le nombre de premiers torons (47) formant la première partie de câble (41).

4. Dispositif d'endoscope (1) selon la revendication 1,
**caractérisé en ce que** la première partie de câble (41) est formée en enroulant des premiers torons (51) et présente un premier diamètre de câble (d3), et
la deuxième partie de câble (42) est formée de seulement un deuxième toron (52) et présente un deuxième diamètre de câble (d4) égal au premier diamètre de câble (d3).
